# EUROPEAN PATENT APPLICATION

(11) **EP 3 622 961 A1**
(43) Date of publication of application: **18.03.2020**
(21) Application number: 18797557.8
(22) Date of filing: 09.05.2018
(51) Int. Cl.: A61K 35/30, A61K 31/522, A61K 31/65, A61K 35/545, A61K 45/00, A61K 48/00, A61P 25/00, A61P 35/00, A61P 43/00, C12N 15/38

(54) **CELL FORMULATION FOR TREATING BRAIN TUMOR**

(30) Priority: 09.05.2017 JP 2017092973
(71) Applicant: Keio University, Tokyo 108-8345 (JP)
(72) Inventor: TODA, Masahiro, Tokyo 160-8582 (JP); OKANO, Hideyuki, Tokyo 160-8582 (JP); MIYOSHI, Hiroyuki, Tokyo 160-8582 (JP); TAMURA, Ryota, Tokyo 160-8582 (JP)
(74) Representative: J A Kemp LLP
(86) International application number: PCT/JP2018/017888
(87) International publication number: WO 2018/207808

(57) **Abstract**

A cell preparation for treating brain tumors used in combination with a prodrug of a drug that kills, or inhibits proliferation of, tumor cells, wherein the cell preparation comprises neural stem cells derived from iPS cells or ES cells having a suicide gene, and the prodrug is a prodrug activated with an enzyme produced by expression of the suicide gene comprised in the neural stem cells is provided to solve the difficulty of obtaining neural stem cells, which is problematic, and to establish new means for treating brain tumors using neural stem cells.

## Description

### Technical Field

The present invention relates to a cell preparation for treating brain tumors and a method for preparing neural stem cells used for the cell preparation.

### Background Art

Malignant gliomas are very difficultly treated in the present therapies due to treatment resistance by brain tumor stem cells (BTSCs), which infiltrate the brain substance widely. Since suicide gene therapies using viral vectors (HSVtK) exerted bystander effects, the effectiveness was expected. Since the diffusion among infiltrating tumor cells was insufficient, the results of the clinical tests were however limited. It has been revealed recently that neural stem cells (NSCs) have the properties of migrating and accumulating in brain tumor stem cells, and attempts to apply neural stem cells to suicide gene treatment as cell vehicles attract attention. It was however difficult in view of problems including an ethical problem to obtain an adequate amount of neural stem cells which can be administered to humans. Then, a technique as to suicide gene treatment using mesenchymal stem cells, which has properties similar to neural stem cells has been developed (Patent Literature 1).

### Citation List

### Patent Literature

Patent Literature 1:
Japanese Patent Laid-Open No. 2006-345726

### Summary of Invention

### Technical Problem

Patent Literature 1 discloses that mesenchymal stem cells derived from rat marrow cells exhibited a higher bystander effect than neural stem cells collected from the rat cerebral cortex (Examples 2 and 3). Although a suicide gene therapy thus using the mesenchymal stem cells is expected to exhibit a high therapeutic effect, it is considered that the administration of the mesenchymal stem cells to the human brain has problems with safety and the like.

Meanwhile, although neural stem cells are safer than the mesenchymal stem cells, it is very difficult to obtain neural stem cells as mentioned above.

Objects of the present invention is to solve the difficulty of obtaining neural stem cells, which is problematic, in such a context and to provide new means for treating brain tumors using neural stem cells.

### Solution to Problem

The present inventor has earnestly examined repeatedly to solve the above-mentioned problem and consequently found that inducing the differentiation of neural stem cells from iPS cells enables obtaining neural stem cells in an amount enough to be used for a suicide gene therapy. The present inventor has also found that when the herpes simplex virus thymidine kinase gene, which is a typical suicide gene, is introduced into iPS cells, and this gene is expressed to cause cytotoxicity. The present inventor has also found that regulating herpes simplex virus thymidine kinase gene expression with doxycycline enables solving this problem with cytotoxicity.

The present invention has been completed based on the above knowledge.

That is, the present invention provides the following [1] to [10].
[1] A cell preparation for treating brain tumors used in combination with a prodrug of a drug that kills, or inhibits proliferation of, tumor cells, wherein the cell preparation comprises neural stem cells derived from iPS cells or ES cells having a suicide gene, and the prodrug is a prodrug activated with an enzyme produced by expression of the suicide gene comprised in the neural stem cells.
[2] The cell preparation for treating brain tumors according to [1], wherein the neural stem cells derived from iPS cells or ES cells having a suicide gene are neural stem cells obtained by sequentially performing the following steps (1) and (2):
   (1) introducing a suicide gene into iPS cells or ES cells,
   (2) differentiating the iPS cells or the ES cells into neural stem cells.
[3] The cell preparation for treating brain tumors according to [2], wherein the suicide gene is a gene structure, expression of which can be regulated artificially.
[4] The cell preparation for treating brain tumors according to [3], wherein the suicide gene structure is a gene structure expressed by addition of doxycycline.
[5] The cell preparation for treating brain tumors according to [1], wherein the neural stem cells derived from iPS cells or ES cells having a suicide gene are neural stem cells obtained by sequentially performing the following steps (1) and (2):
   (1) differentiating iPS cells or ES cells into neural stem cells,
   (2) introducing a suicide gene into the neural stem cells.
[6] The cell preparation for treating brain tumors according to any one of [1] to [5], wherein the suicide gene is a herpes simplex virus thymidine kinase gene, and the prodrug is ganciclovir.
[7] A method for preparing neural stem cells having a suicide gene, comprising performing the following steps (A1) and (A2) or steps (B1) and (B2) sequentially:
   (A1) introducing a suicide gene into iPS cells or ES cells,
   (A2) differentiating the iPS cells or the ES cells into neural stem cells,
   (B1) differentiating iPS cells or ES cells into neural stem cells,
   (B2) introducing a suicide gene into the neural stem cells.
[8] The method for preparing neural stem cells having a suicide gene according to [7], wherein the suicide gene in the step (A1) is a gene structure, expression of which can be regulated artificially.
[9] The method for preparing neural stem cells having a suicide gene according to [8], wherein the suicide gene structure in the step (A1) is a gene structure expressed by addition of doxycycline.
[10] The method for preparing neural stem cells having a suicide gene according to any one of [7] to [9], wherein the suicide gene is a herpes simplex virus thymidine kinase gene.

The present invention provides the following [A].
[A] A method for treating brain tumors, comprising the following steps (1) and (2):
   (1) administering a cell preparation to a patient, wherein the cell preparation comprises neural stem cells derived from iPS cells or ES cells having a suicide gene,
   (2) administering a prodrug to the patient, wherein the prodrug is a prodrug of a drug that kills, or inhibits proliferation of, tumor cells, and the prodrug is activated with an enzyme produced by expression of the suicide gene comprised in the neural stem cells.

The present specification includes the contents described in the specification and/or drawings of the Japanese Patent Application No. 2017-92973, which is the basis of the priority of the present application.

### Advantageous Effects of Invention

The present invention provides new means for treating brain tumors using neural stem cells derived from iPS cells or ES cells.

### Brief Description of Drawings

[Figure 1] Figure 1 shows the summary of a suicide gene cell therapy for malignant brain tumors using therapeutic stem cells (TSCs) expressing HSVtk.
[Figure 2] Figure 2 shows the results of the luminescence imaging analysis (IVIS) of tumors. Stem cells expressing HSVtk were used as therapeutic stem cells.
[Figure 3] Figure 3 shows the measurement results of the ROI values of the luminescence imaging. Stem cells expressing HSVtk were used as therapeutic stem cells.
[Figure 4] Figure 4 shows HE-stained images (left) and Venus fluorescence images (right) of mouse brain sections. Stem cells expressing HSVtk were used as therapeutic stem cells.
[Figure 5] Figure 5 shows the results of the analysis of tumor volumes. Stem cells expressing HSVtk were used as therapeutic stem cells.
[Figure 6] Figure 6 shows the results of the survival analysis of the transplanted TSCs. Stem cells expressing HSVtk were used as therapeutic stem cells. The upper row shows the brains of an untreated group, and the lower row shows the brains of a treated group. From the left, images are bright fields, images stained with Venus (U87 tumor cells are stained), images stained with KO (stem cells are stained), images stained with DAPI (nuclei are stained), and a merge (fused image of Venus, KO and DAPI).
[Figure 7] Figure 7 shows the results of the survival analysis of brain tumor models. Stem cells expressing HSVtk were used as therapeutic stem cells.
[Figure 8] Figure 8 shows the summary of a suicide gene cell therapy using Tet-induced therapeutic stem cells (TSCs) expressing HSVtk.
[Figure 9] Figure 9 shows the result of the luminescence imaging analysis (IVIS) of tumors. Tet-induced stem cells expressing HSVtk were used as therapeutic stem cells.
[Figure 10] Figure 10 shows the results of the survival analysis of brain tumor models. Tet-induced stem cells expressing HSVtk were used as therapeutic stem cells.
[Figure 11] Figure 11 shows HE-stained images (left) and Venus fluorescence images (right) of mouse brain sections. Tet-induced stem cells expressing HSVtk were used as therapeutic stem cells.
[Figure 12] Figure 12 shows the results of the analysis of tumor volumes. Tet-induced stem cells expressing HSVtk were used as therapeutic stem cells.

### Description of Embodiments

The present invention will be described in detail hereinafter.

A cell preparation for treating brain tumors of the present invention is characterized in that the cell preparation is used in combination with a prodrug of a drug that kills, or inhibits proliferation of, tumor cells, wherein the cell preparation comprises neural stem cells derived from iPS cells or ES cells having a suicide gene, and the prodrug is a prodrug activated with an enzyme produced by expression of the suicide gene comprised in the neural stem cells.

The "brain tumors" in the present invention means tumors which are developed in intracranial tissue. Specific examples thereof include gliomas, medulloblastomas, neuroblastomas, meningiomas, pituitary adenomas, neurinomas, primary central nervous system lymphomas, sarcomas and spinal cord tumors. In the present invention, although all of these brain tumors can be objects to be treated, it is preferable that gliomas be objects to be treated.

The "treatment" in the present invention means not only killing tumor cells but also reducing tumor cells or inhibiting the proliferation of tumor cells.

The "suicide gene" in the present invention means a gene which is expressed in cells and can kill the cells. As the suicide gene, a metabolic toxic gene can be used. Suitable specific examples thereof include the herpes simplex virus thymidine kinase (HSVtk) gene (Proc. Natl. Acad. Sci, USA 78 (1981) 1441-1445). Examples of suicide genes other than the herpes simplex virus thymidine kinase gene include a cytosine deaminase gene, a uracil phosphoribosyl transferase gene, a guanine phosphoribosyl transferase gene and a nitroreductase gene.

As long as the suicide gene can be expressed at the time of treatment, the suicide gene is not particularly limited. As mentioned above, since some suicide genes exhibit cytotoxicity in iPS cells (for example, HSVtk gene), it is preferable that the suicide gene be a gene structure, the expression of which can be regulated artificially. Using the gene structure containing such a suicide gene, the expression of which can be regulated artificially, enables suppressing the expression of the suicide gene in iPS cells and inducing the expression of the suicide gene at the time of the treatment of brain tumors. A problem with cytotoxicity can be solved. Such a gene structure can be produced using a sequence which enables regulating the expression of the suicide gene artificially as mentioned below.

As long as the prodrug in the present invention is a prodrug of a drug that kills, or inhibits proliferation of, tumor cells, and the prodrug is activated with an enzyme produced by expression of the suicide gene and comes to exhibit the effect of killing tumor cells or inhibiting the proliferation thereof, the prodrug may be any prodrug. A prodrug to be used can be determined depending on the suicide gene. For example, when the suicide gene is a herpes simplex virus thymidine kinase gene, ganciclovir (GCV), aciclovir, penciclovir, adefovir, tenofovir or the like can be used as the prodrug. When the suicide gene is a cytosine deaminase gene, 5-fluorocytosine or the like can be used as the prodrug. When the suicide gene is an uracil phosphoribosyltransferase gene, 5-fluorouracil or the like can be used as the prodrug. When the suicide gene is a guanine phosphoribosyltransferase gene, 6-thioxanthine, 6-thioguanine or the like can be used as a prodrug. When the suicide gene is a nitroreductase gene, CB1954 or the like can be used as the prodrug.

The "neural stem cells" in the present invention means stem cells having the capability to supply cells which differentiates into neurons and glia cells. The cell preparation of the present invention contains these neural stem cells. As long as the cell preparation does not exert a great adverse influence on the effect of treating brain tumors, the cell preparation may contain cells other than neural stem cells. When neural stem cells are prepared from iPS cells in accordance with a method described in the below-mentioned Mol Brain 9: 85, 2016, not only neural stem cells but also neural precursor cells are produced. For this reason, the cell preparation of the present invention usually contains both neural stem cells and neural precursor cells. Neural stem cells used in the present invention is neural stem cells derived from pluripotent stem cell such as iPS cells, and neural stem cells collected from the brain or the like is not used as they are.

Neural stem cells derived from iPS cells having a suicide gene can be prepared by sequentially performing steps (A1) and (A2) or steps (B1) and (B2) described below.

In the step (A1), a suicide gene is introduced into iPS cells.

As long as iPS cells to be used can be differentiated into neural stem cells, the origin thereof, a reprogramming factor to be introduced, a method for introducing a reprogramming factor and the like are not particularly limited. Since the cell preparation of the present invention is used mainly for treating human brain tumors, it is however preferable to use iPS cells derived from a human in such a case. In this case, iPS cells derived from a patient to whom the cell preparation is administered may be used, and iPS cells derived from a human other than the patient may be used. As mentioned above, although a method for introducing a reprogramming factor is not limited, either, it is preferable to use integration-free iPS cells. Although iPS cells to be used may be prepared in accordance with a well-known method, iPS cells can also be obtained from research institutions such as the Center for iPS Cell Research and Application (CiRA), Kyoto University. Examples of suitable iPS cell lines include 1210B2 which the CiRA has.

Examples of the method of introducing a suicide gene into iPS cells include a method of introducing a vector in which a suicide gene is incorporated. It is preferable to use viral vectors such as adenovirus vectors, adeno-associated viral vectors, retroviral vectors and lentiviral vectors as the vector. Among these, a vector which incorporates a suicide gene into a genome is preferable, and lentiviral vectors are the most preferable.

A sequence which enables regulating the expression of a suicide gene artificially is preferably included in the vector besides the suicide gene. It is preferable to use the tetracycline induction system, which is a well-known expression induction system (PLoS One 8: e59890, 2013), as such a sequence. This tetracycline induction system comprises a region which expresses a reverse tetracycline-controlled transactivator (rtTA), a gene of interest and a region including a TRE promoter disposed upstream thereof. The rtTA bonds to the TRE promoter by bonding to tetracycline and induces the expression of the gene of interest disposed downstream. Using this system enables inducing the expression of the suicide gene by the addition of tetracycline. Although the expression in this system can also be induced with tetracycline as mentioned above, but is usually induced with doxycycline, which is a derivative of tetracycline. The sequence which enables regulating the expression of the suicide gene artificially is not limited to this tetracycline induction system, and various inducible promoters such as medicine inducible promoters may be used.

Sequences such as a reporter gene, IRES and the like may be included in the vector besides the suicide gene or the sequence which enables regulating the expression of the suicide gene artificially.

The suicide gene can be introduced into iPS cells by a vector in accordance with a well-known method depending on the type of vector to be used.

In the step (A2), iPS cells are differentiated into neural stem cells.

Differentiation from iPS cells to neural stem cells may be performed in accordance with either well-known method of a method for preparation through embryoid bodies and a method for preparation without forming embryoid bodies. For example, the differentiation can be performed in accordance with a method described in Mol Brain 9:85, 2016.

According to the above-mentioned method, embryoid bodies can be formed from iPS cells using well-known embryoid body-forming medium, and the embryoid body medium contains a TGFβ family inhibitor (for example, SB431542) and a BMB inhibitor (for example, LDN-193189).

Differentiation from the embryoid bodies to neural stem cells can be performed using well-known neurosphere medium. For example, the neurosphere medium contains an epidermal growth factor and a fibroblast growth factor-2, a leukemia inhibitory factor, a B-27 supplement and the like. Culturing the embryoid bodies in the neurosphere medium forms cell masses called neurospheres and containing neural stem cells. The formed neurospheres are collected and disassembled into single cells. The single cells are cultured in neurosphere medium to form neurospheres again. Such operation is repeated several times, and neural stem cells can be obtained by collecting neurospheres.

When neural stem cells derived from iPS cells having a suicide gene are prepared by sequentially performing the steps (A1) and (A2), there is the advantage of neural stem cells for treatment being able to be supplied steadily by proliferating iPS cells.

In the step (B1), iPS cells are differentiated into neural stem cells.

The differentiation from iPS cells to neural stem cells in the step (B1) can be performed in the same way as the differentiation in the step (A2).

In the step (B2), a suicide gene is introduced into neural stem cells.

The introduction of a suicide gene into neural stem cells in a step (B2) can be performed in the same way as the introduction in the step (A1). Since a problem with cytotoxicity may occur in the step (A1), a sequence which enables regulating the expression of a suicide gene artificially is however included in a vector preferably. Since such a problem occurs very rarely in the step (B2), a sequence which enables regulating the expression of a suicide gene artificially is, however, usually unnecessary.

As mentioned above, neural stem cells having a suicide gene can be prepared from iPS cells by sequentially performing the steps (A1) and (A2) or the steps (B1) and (B2). iPS cells and ES cells have the same nature. Even though ES cells are used instead of iPS cells, neural stem cells having a suicide gene can therefore be prepared.

As mentioned above, "neural stem cells," which are a "product," are not specified by structure or properties but specified by a preparation method herein. This is because since cells are parts of the living body, the structure and properties thereof are very complicated, and markedly excessive economic expenditure and time are taken to work to specify them.

The cell preparation of the present invention may contain other pharmaceutically acceptable components besides neural stem cells. Examples of such other components include carriers, excipients, disintegrators, buffers, emulsifiers, suspending agents, soothing agents, stabilizers, preservatives, antiseptics and physiological saline solutions. Dimethyl sulfoxide, serum albumin or the like may be contained to protect cells at the time of freeze storage, and an antibiotic or the like may be contained to prevent the contamination and proliferation of bacteria.

The number of neural stem cells contained in the cell preparation of the present invention can be optionally determined in view of the sex, age or weight of a subject, the state of an affected part, the state of cells to be used or the like so as to obtain a desired effect (for example, the disappearance of tumors or the reduction of tumor size) in the treatment of brain tumors.

The cell preparation of the present invention may be administered a plurality of times (for example, 2 to 10 times) at intervals (for example, twice per day, once per day, twice per week, once per week, once per two week). Although the dose can be optionally determined in view of the sex, age or weight of a subject, the state of an affected part, the state of cells to be used or the like, the dose is preferably 1 × 10⁴ to 1 × 10⁶ cells per individual (human) 1 to 10 times.

The administration site or administration method of the cell preparation of the present invention is not particularly limited. Examples of the administration method include tumor local administration, intracarotidarterial administration and intravenous administration.

The administration site or administration method of a prodrug used in combination with the cell preparation of the present invention is not particularly limited, either. Examples of the administration method also include intraperitoneal administration besides tumor local administration, intracarotidarterial administration and intravenous administration mentioned above.

The prodrug may be administered before or after the administration of the cell preparation of the present invention or at the same time as the administration of the cell preparation, but is usually administered in divided doses after the administration of the cell preparation. The dose of the prodrug can be optionally determined in view of the type of prodrug to be used, the sex, age or weight of a subject or the state of an affected part. When ganciclovir is administered, ganciclovir is preferably administered at 3 to 5 mg/kg per once, twice per day for 2 to 3 weeks (28 times to 42 times) per individual (human).

When the tetracycline induction system is used to prevent cytotoxicity, doxycycline is also administered at the time of treatment to express a suicide gene. Examples of a method for administer doxycycline include oral administration, tumor local administration, intracarotidarterial administration, intravenous administration and intraperitoneal administration. Doxycycline may be administered before or after the administration of the cell preparation of the present invention or at the same time as the administration of the cell preparation, but is usually administered after the administration of the cell preparation.

### Examples

Next, although the present invention will be described in more detail by giving Examples, the present invention is not limited to these Examples.

### (A) Material and method

### <Human iPS cell>

The used human iPS cell (1210B2) was obtained from the Center for iPS Cell Research and Application (CiRA), Kyoto University. Then, 1210B2 was established by a method for introducing a reprogramming factor using an episomal vector for human peripheral blood mononuclear cells (Stem Cells 31: 458-66, 2013). Human iPS cells were inoculated on a plastic culture dish coated with iMatrix-511 (produced by Nippi, Incorporated) and subjected to maintenance culture using Stem Fit AK03 or AK03N medium (produced by AJINOMOTO CO., INC.) by a known feeder-free method (Sci Rep 4: 3594, 2014).

### <Differentiation induction from human iPS cells to neural stem/ precursor cells (NS/PCs)>

Differentiation induction from human iPS cells to NS/PCs was performed by a known method (Mol Brain 9: 85, 2016). First, a 10 µM ROCK inhibitor Y276352 (produced by Wako Pure Chemical Corporation) was added to medium of iPS cells, the iPS cells were incubated for 1 to 3 hours, then washed with PBS and disassembled into single cells using TrypLE Select (produced by Life Technologies Corporation). The human iPS cells disassembled into the single cells were suspended in EB (embryoid body) - forming medium to which a 10 µM ROCK inhibitor Y276352 was added (10 µM SB431542 and 100 nM LDN-193189 were added to Stem Fit medium to which C liquid was not added), inoculated at a concentration of 9.0 × 10³ cells/75 µl per 1 well of a low adhesive 96 hole-culture plate (Prime Surface 96V, manufactured by Sumitomo Bakelite Co., Ltd.) and cultured. Then, 75 µl of EB-forming media was added 1 day after, a half amount of EB-forming medium was replaced everyday on and after, and the human iPS cells were cultured for 13 to 14 days to obtain EBs. The EBs were collected from each well and cultured in NS (neurosphere) medium (to D-MEM/Ham's F-12 medium (containing HEPES) (produced by Wako Pure Chemical Corporation) were added a 20 ng/ml recombination human epidermal growth factor (produced by PeproTech, Inc.), a 20 ng/ml recombination human fibroblast growth factor-2 (produced by PeproTech, Inc.), a 10³units/ml recombination human leukemia inhibitory factor (produced by NACALAI TESQUE, INC.), a 2% B-27 supplement (produced by Thermo Fisher Scientific K.K.) and 1 unit/ml heparin sodium (produced by AY PHARMACEUTICALS CO., LTD.)) for 7 days. The medium was replaced once per 3 or 4 days during culture. Cell masses were collected by a centrifuge, disassembled into single cells using TrypLE Select and suspended in NS medium. The single cells were inoculated into a low adhesive flask (manufactured by Corning Incorporated) at a concentration of 1 × 10⁵ cells/ml and cultured for 10 days with the medium replaced every 3 to 4 days to obtain primary NS/PCs. Similarly, the primary NS/PCs were collected by the centrifuge, disassembled into single cells using TrypLE Select and suspended in NS medium. The single cells were inoculated into a low adhesive flask at a concentration of 1 × 10⁵ cells/ml and cultured for 7 to 10 days with the medium replaced every 3 to 4 days to obtain secondary, tertiary, quaternary and quinary NS/PCs.

### <Production of HSVtk expression lentiviral vector>

The cDNA of the Humanized, CpG-free HSVtk gene (HSV1tk) wherein the cDNA was subcloned from the pSelect-zeo-HSV1tk plasmid (produced by InvivoGen Corporation) to pENTR/D-TOPO (produced by Thermo Fisher Scientific K.K.) by PCR and the nucleotide sequence of the cDNA was confirmed, was used. The HSV1tk cDNA was subcloned to the lentiviral vector plasmids CSII-EF-RfA-IRES2-hKO1 and CSIV-RfA-TRE-EF-KT to obtain CSIV-EF-HSV1tk-IRES2-hKO1 and CSIV-HSV1tk-TRE-EF-KT. CSIV-EF-HSV1tk-IRES2-hKO1 was used to produce a lentiviral vector which expresses HSV1tk and hKO1 (Humanized Kusabira-Orange fluorescence protein gene) under the EF-1α promoter. CSIV-HSV1tk-TRE-EF-KT was used to produce a lentiviral vector which expresses hKO1 and rtTA (reverse Tet-controlled transactivator) under the EF-1α promoter and expresses HSV1tk under the tetracycline (Tet)-induced promoter. The lentiviral vectors were produced by a known method (J Virol 72: 8150-8157, 1998; PLoS One 8: e59890, 2013; Sci Rep 5: 10434, 2015).

### <Preparation of human glioma cell line U87 expressing ffLuc>

The human glioma cell line U87 was infected with the ffLuc (fusion gene of Venus fluorescence protein and Luc2 firefly luciferase) expression lentiviral vector (CSII-EF-ffLuc) (Cell Transplant 20:727-739, 2011). The clones were sorted with a cell sorter to obtain the U87 cell line, which expresses ffLuc highly and steadily.

### <Preparation of NS/PCs expressing HSVtk>

Secondary or tertiary NS/PCs differentiation-induced from human iPS cells were collected with the centrifuge and disassembled into single cells using TrypLE Select. The single cells were suspended in NS medium, inoculated into a low adhesive flask at a concentration of 1 × 10⁵ cells/ml, infected with the CSIV-EF-HSV1tk-IRES2-hKO1 lentiviral vector at an MOI (multiplicity of infection) of 0.5 to 1, and cultured for 7 days with the medium replaced every 3 to 4 days. The obtained NS/PCs were infected with the CSIV-EF-HSV1tk-IRES2-hKO1 lentiviral vector at an MOI of 0.5 to 1 in the same way again to prepare therapeutic stem cells (TSCs), that is, NS/PCs expressing HSVtk. It was confirmed that the cell death of the NS/PCs expressing HSVtk was induced by adding 1 µg/ml ganciclovir (GCV) to the medium.

### <Examination of therapeutic effect of NS/PCs expressing HSVtk in brain tumor model mice>

First, 1 × 10⁵ cells/2 µl U87 cells expressing ffLuc and 5 × 10⁵ cells/2µl NS/PCs expressing HSVtk were mixed and stereotactically transplanted to the right striate body (2 mm away from the anterior fontanel to the right and 3 mm deep from the brain surface) of each T cell-deficient mouse (female BALB/c nude mouse, 20g, 6w) subjected to general anesthesia.

Then, 50 mg/kg of GCV was intraperitoneally administered to the treated group twice per day for 21 days from the day following the transplantation (group 1: HSVtk (+)/ GCV (+), n = 10). Next, 200 µl of 1 × PBS was intraperitoneally administered to the control group for the same period (group 2: HSVtk (+)/ GCV (-), n = 10).

Change in the tumor of the same individual over time was observed with an IVIS, that is, an in vivo imaging system, every week. At the time of photographing with IVIS, 200 µl of 30mg/ml VivoGlo (TM) Luciferin was intraperitoneally administered to the individual subjected to isoflurane inhalation anesthesia, and the tumor was photographed for 10 minutes when the peak was reached.

Four mice of each of both groups were decapitated in the third week, and the actual tumor volumes were measured. The mice were decapitated by perfusing the mice with 4% paraformaldehyde for fixation through the heart. The brain tissues taken out were replaced with 10% and 20% sucrose, and the brain tissues were sliced to a thickness of 20 µm. An 8-well dish the wells of which each contain 1 ml of an antifreeze solution was charged with the slices sequentially and stored at -20°C.

The brain slices were observed by staining with HE or through a fluorescence microscope since U87 expresses Venus fluorescence protein to measure the tumor volumes in more detail. It was evaluated whether the transplanted TSCs remained by fluorescently staining the brain tissues of both groups with an anti-KO antibody in the third week in the same way. Finally, the survival curves of both groups were compared.

### <Preparation of tetracycline (Tet)-induced NS/PCs expressing HSVtk>

Human iPS cells were infected with the CSIV-HSV1tk-TRE-EF-KT lentiviral vector at an MOI of 2.5 to 10 and cultured. The clones were then sorted with the cell sorter to obtain an iPS cell line expressing hKO1 steadily and highly. It was confirmed that the cell death of Tet-induction iPS cells expressing HSVtk was induced by adding 1 µg/ml doxycycline (Dox) and 1 µg/ml GCV to the medium.

Tet-induced iPS cells expressing HSVtk were differentiation-induced into NS/PCs to obtain secondary and tertiary Tet-induced NS/PCs expressing HSVtk. It was confirmed that the cell death of these Tet-induction NS/PCs expressing HSVtk was also induced by adding 1 µg/ml doxycycline (Dox) and 1 µg/ml GCV to the medium. <Examination of the therapeutic effect of Tet-induced NS/PCs expressing HSVtk in brain tumor model mice>

Three groups which are a treated group 1 (Tet-HSVtk (+)/ Dox (+)/GCV (+), n = 12), a control group 2 (Tet-HSVtk (+)/ Dox (-)/ GCV (+), n = 8), and a control group 3 (Tet-HSVtk (-) / Dox (+)/ GCV (+), n= 4) were produced. All of the method for evaluating tumors, the survival curve analysis and the like were performed in the same way in the above-mentioned Examination of therapeutic effect of NS/PCs expressing HSVtk (Feed containing 200 mg/kg of Dox was orally ingested).

### (B) Results

### (1) Suicide gene cell therapy for malignant brain tumors using therapeutic stem cells expressing HSVtk

### <Summary>

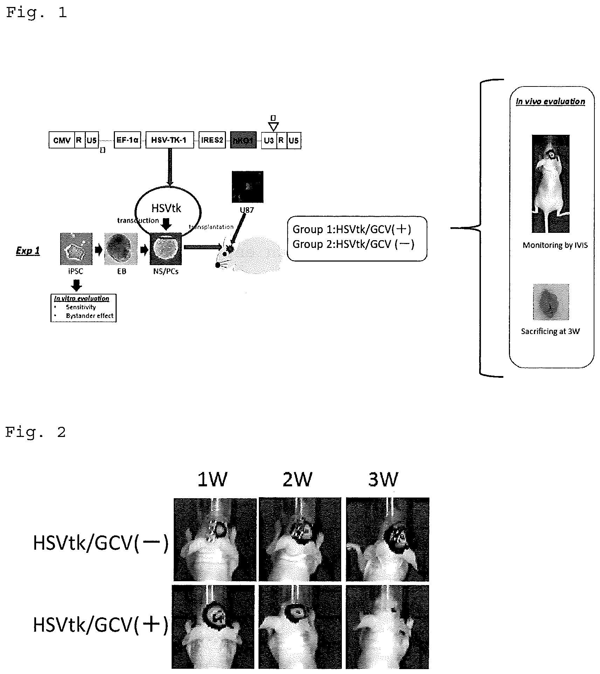

Figure 1 shows the summary of a suicide gene cell therapy for malignant brain tumors using therapeutic stem cells expressing HSVtk. Induced pluripotent stem (iPS) cells were differentiation-induced into neural stem cells (NSCs). The herpes simplex virus thymidine kinase (HSVtk) gene, which is a suicide gene, was then introduced with the lentiviral vector. Therapeutic stem cells (TSCs) expressing HSVtk were established. A mixture of 1 × 10⁵ human glioma cells (U87) and 5 × 10⁵ TSCs was transplanted to the T cell-deficient mouse brain (right striate body). In a treated group, 50 mg/kg of ganciclovir (GCV) was administered twice per day for 21 days from the day following the transplantation, and PBS was administered to a control group. The survival analysis was performed while the luminescence imaging analysis (IVIS) of tumors was performed chronologically. Some mice were decapitated in the third week after the transplantation, and the histological analysis of the brains was performed.

### <Chronological analysis of tumors by luminescence imaging analysis (IVIS)>

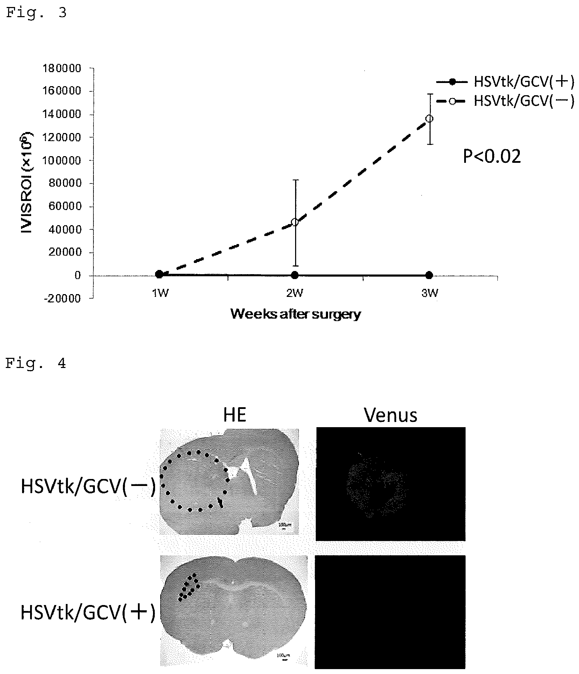

Tumors tended to be smaller in the treated group (GCV+) than in the control group (GCV-) (Figure 2). When the ROI values of the luminescence imaging was measured, the ROI values of the treated group were significantly low, and the ROI values were remarkably low especially from the second week onward (Figure 3). (P = 0.02)

### <Histological analysis of brain tumor model mice (in the third week after the transplantation of glioma cells and TSCs: n = 4)>

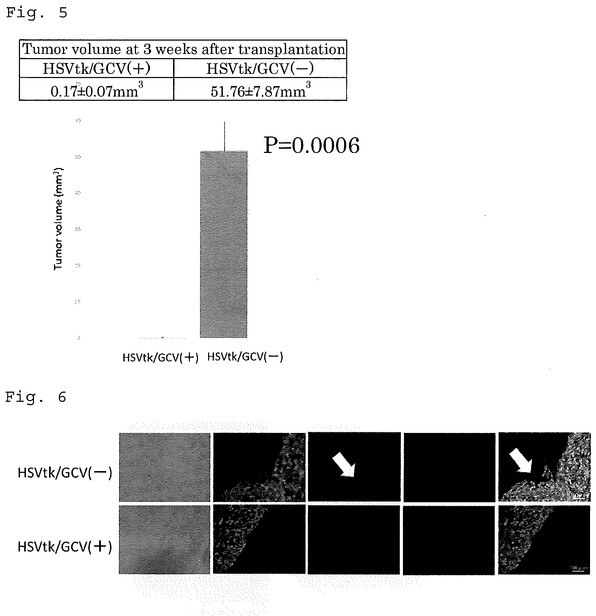

Since the Venus fluorescence protein gene was introduced into the glioma cells (U87), the tumor volumes were quantitatively analyzed using the fluorescence microscope (Figure 4). The tumor volume of a treated group (GCV+) was 0.17 ± 0.07 mm³, and was significantly small as compared with 51.76 ± 7.87 mm³ of a control group (GCV-) (Figure 5). (P = 0.0006)

The extinction of the above-mentioned transplanted TSCs in the mouse brains was confirmed as to the safety of the present therapy (the risk of the tumorigenesis of iPS cells). Since the Kusabira-Orange(KO) fluorescence protein gene was introduced into the TSCs, the TSCs were analyzed by fluorescence staining using an anti-KO antibody. Consequently, although the transplanted TSCs remained in the brains in the control group (GCV-), clear survival of the transplanted TSCs was not observed in the treated group (GCV+) (Figure 6). It was therefore confirmed that the transplanted TSCs died within three weeks by the HSVtk/GCV system (each n = 4).

### <Survival analysis of brain tumor model>

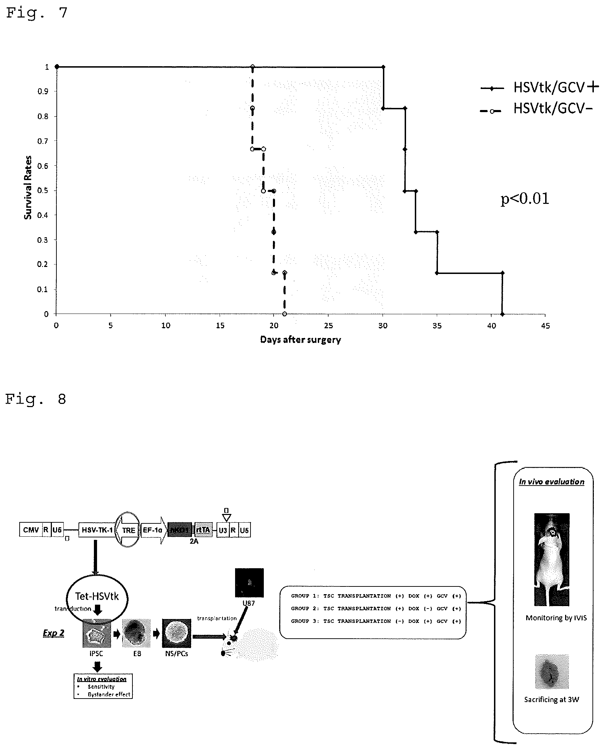

In the treated group (GCV+), significant extension of the survival period was observed as compared with the control group (GCV-) (Figure 7). The average survival period of the treated group was 4 days, and the average survival period of the control group was 19 days (p < 0.01) .

### (2) Suicide gene cell therapy using Tet-induced therapeutic stem cells (TSCs) expressing HSVtk

### <Summary>

Figure 8 shows the summary of a suicide gene cell therapy using Tet-induced therapeutic stem cells (TSCs) expressing HSVtk. Although it was attempted to introduce the HSVtk gene into human iPS cells for supplying the cells steadily for clinical application, an iPS cell line expressing HSVtk could not be established due to cytotoxicity. Then, a Tet-induced iPS cell line expressing HSVtk was established using the tetracycline (Tet)-induced promoter which could regulate gene expression. The cell line could be differentiated into neural stem cells (NS/PCs), and Tet-induced therapeutic stem cells (TSCs) expressing HSVtk could be steadily supplied.

In the Tet-induced HSVtk, only when doxycycline (Dox) is added, the HSVtk gene is expressed.

### <Luminescence imaging analysis (IVIS) and survival analysis of tumors>

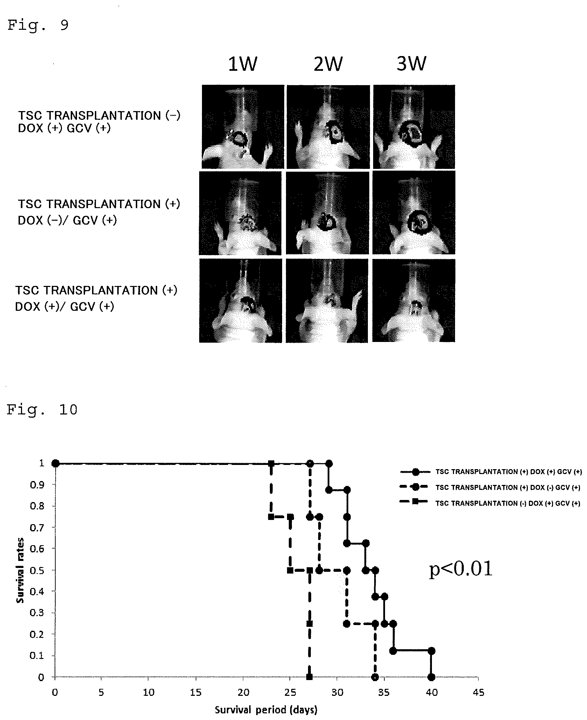

In IVIS analysis, the reduction of the tumors was observed in the treated group under the conditions of TSC transplantation (+)/ Dox (+)/ GCV (+) as compared with the control group under the conditions of TSC transplantation (-)/ Dox (+)/ GCV (+) and the control group under the conditions of TSC transplantation (+) / Dox (-) / GCV (+) (Figure 9).

In survival analysis, significant extension of the survival period was observed in the treated group under the conditions of TSC transplantation (+) /Dox (+)/ GCV (+) as compared with the control group under the conditions of TSC transplantation (-)/ Dox (+)/ GCV (+) (Figure 10). The average survival period of the treated group was 34 days, and the average survival period of the control group was 25.5 days (p < 0.01). (Meanwhile, the statistically significant difference was not observed between the control group under the conditions of TSC transplant (+)/ Dox (-)/ GCV (+) and the treated group.) In the Tet-induced HSVtk, only when doxycycline (Dox) is added, the HSVtk gene is expressed.

### <Histological analysis of brain tumor model mice (third week after glioma cells and TSCs transplantation: n = 4)>

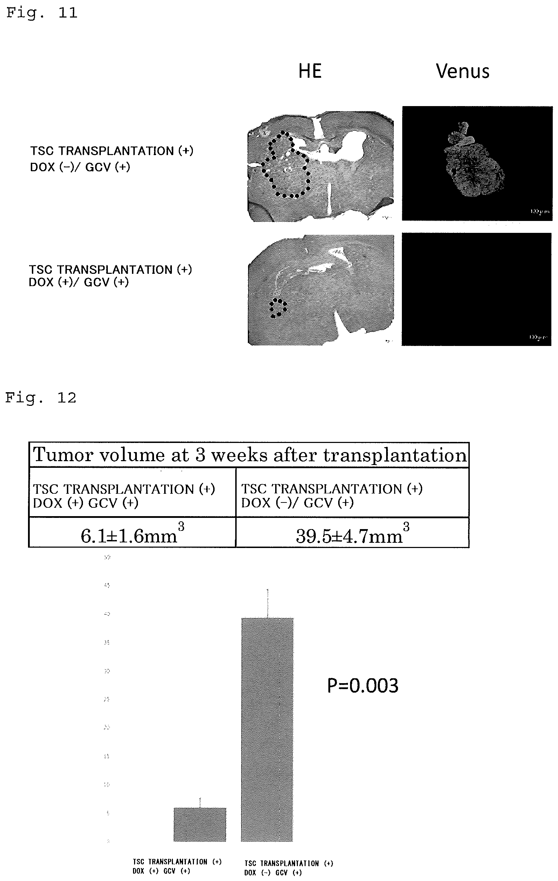

As compared with the control group under the conditions of TSC transplantation (+)/ Dox (-)/ GCV (+), a significant effect of reducing tumors was observed in the treated group under the conditions of TSC transplantation (+)/ Dox (+)/ GCV (+) (Figures 11 and 12). (p = 0.003)

All of the publications, patents and patent applications cited herein are incorporated herein as reference as they are.

### Industrial Applicability

Since a cell preparation for treating brain tumors of the present invention can be used as a pharmaceutical, the present invention can be used in industrial fields such as medicine manufacture.

## Claims

1. A cell preparation for treating brain tumors used in combination with a prodrug of a drug that kills, or inhibits proliferation of, tumor cells, wherein the cell preparation comprises neural stem cells derived from iPS cells or ES cells having a suicide gene, and the prodrug is a prodrug activated with an enzyme produced by expression of the suicide gene comprised in the neural stem cells.

2. The cell preparation for treating brain tumors according to claim 1, wherein the neural stem cells derived from iPS cells or ES cells having a suicide gene are neural stem cells obtained by sequentially performing the following steps (1) and (2):
(1) introducing a suicide gene into iPS cells or ES cells,
(2) differentiating the iPS cells or the ES cells into neural stem cells.

3. The cell preparation for treating brain tumors according to claim 2, wherein the suicide gene is a gene structure, expression of which can be regulated artificially.

4. The cell preparation for treating brain tumors according to claim 3, wherein the suicide gene structure is a gene structure expressed by addition of doxycycline.

5. The cell preparation for treating brain tumors according to claim 1, wherein the neural stem cells derived from iPS cells or ES cells having a suicide gene are neural stem cells obtained by sequentially performing the following steps (1) and (2):
(1) differentiating iPS cells or ES cells into neural stem cells,
(2) introducing a suicide gene into the neural stem cells.

6. The cell preparation for treating brain tumors according to any one of claims 1 to 5, wherein the suicide gene is a herpes simplex virus thymidine kinase gene, and the prodrug is ganciclovir.

7. A method for preparing neural stem cells having a suicide gene, comprising performing the following steps (A1) and (A2) or steps (B1) and (B2) sequentially:
(A1) introducing a suicide gene into iPS cells or ES cells,
(A2) differentiating the iPS cells or the ES cells into neural stem cells,
(B1) differentiating iPS cells or ES cells into neural stem cells,
(B2) introducing a suicide gene into the neural stem cells.

8. The method for preparing neural stem cells having a suicide gene according to claim 7, wherein the suicide gene in the step (A1) is a gene structure, expression of which can be regulated artificially.

9. The method for preparing neural stem cells having a suicide gene according to claim 8, wherein the suicide gene structure in the step (A1) is a gene structure expressed by addition of doxycycline.

10. The method for preparing neural stem cells having a suicide gene according to any one of claims 7 to 9, wherein the suicide gene is a herpes simplex virus thymidine kinase gene.
